(19) **Europäisches Patentamt**

**European Patent Office**

**Office européen des brevets**

(11) **EP 0 951 897 B1**

(12) **FASCICULE DE BREVET EUROPEEN**

(45) Date de publication et mention
de la délivrance du brevet:
**03.09.2003 Bulletin 2003/36**

(51) Int Cl.7: **A61K 7/48**

(21) Numéro de dépôt: **99400904.1**

(22) Date de dépôt: **14.04.1999**

(54) **Composition à application topique contenant un copolymère d'oléfines à cristallisation contrôlée**

Ein Olefincopolymer mit kontrolierter Kristallisation enthaltende Zusammensetzung zur topischen Anwendung

Topical composition containing an olefinic copolymer with controlled crystallisation

(84) Etats contractants désignés:
**DE ES FR GB IT**

(30) Priorité: **21.04.1998 FR 9804950**

(43) Date de publication de la demande:
**27.10.1999 Bulletin 1999/43**

(73) Titulaire: **L'OREAL**
**75008 Paris (FR)**

(72) Inventeur: **Mondet, Jean**
**93600 Aulnay-sous-Bois (FR)**

(74) Mandataire: **Casalonga, Axel**
**BUREAU D.A. CASALONGA - JOSSE**
**Paul-Heyse-Strasse 33**
**80336 München (DE)**

(56) Documents cités:
**EP-A- 0 081 787**    **EP-A- 0 270 339**
**EP-A- 0 665 008**    **US-A- 5 695 772**

• **BASE DE DONN ES : "CHEMICAL ABSTRACTS" (SERVEUR: STN); abrégé 123: 152 608, Colombus, OH, USA; & JP 07 149 613 A (KAO CORP.) 13 JUIN 1995 XP002091759**
• **H. STARKWEATHER ET AL.: "crystalline order in copolymers of ethylene and propylene" MACROMOLECULES, vol. 15, 1982, pages 1600-1604, XP002091758**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a trait à une composition contenant au moins un copolymère d'oléfines soluble ou dispersable dans une phase grasse, destinée en particulier aux domaines cosmétique, dermatologique, pharmaceutique et hygiénique. Plus spécialement, l'invention se rapporte à une composition pour le soin et/ou le maquillage de la peau aussi bien du visage que du corps humain, des muqueuses comme les lèvres et l'intérieur des paupières inférieures, ou encore des phanères comme les cils, les sourcils, les ongles et les cheveux.

Cette composition peut se présenter notamment sous forme de produit coulé en stick ou en coupelle comme les rouges ou baumes à lèvres, les fonds de teint coulés, les produits anti-cernes, les fards à paupières ou à joues, sous forme de pâte ou de crème plus ou moins fluide comme les fonds de teint ou rouges à lèvres fluides, les eyes-liners, le maquillage du corps, les compositions de protection solaire ou de coloration de la peau, les mascaras, ou de poudres libres ou compactées.

Les produits de maquillage ou de soin de la peau ou des lèvres des êtres humains comme les fonds de teint ou les rouges à lèvres, contiennent généralement des phases grasses telles que des cires et des huiles, des pigments et/ou charges et, éventuellement des additifs comme des actifs cosmétiques ou dermatologiques. Elles peuvent aussi contenir des produits dits "pâteux", de consistance souple, permettant d'obtenir des pâtes, colorées ou non, à appliquer au pinceau.

De telles compositions contenant des cires sont décrites par exemple dans le brevet japonais JP-A-07 149 613 et dans la demande de brevet EP 0 665 008.

Le brevet japonais JP-A-07 149 613 et le résumé correspondant, publié dans Chemical Abstracts, abrégé 123 : 152608, divulguent des compositions cosmétiques contenant (A) une cire copolymère d'éthylène et de propylène ayant un point de fusion de 75 - 120 °C et une masse moléculaire comprise entre environ 200 et 1500, et (B) des hydrocarbures linéaires ou des cires de paraffines ayant un point de fusion compris entre 60 et 120 °C.

La demande de brevet EP 0 665 008 divulgue des compositions cosmétiques ou dermatologiques anhydres contenant l'association d'une huile de silicone et d'une cire d'un homopolymère ou copolymère d'éthylène ayant une masse moléculaire comprise entre 200 et 1500.

La demande de brevet EP 0 270 339 divulgue des compositions topiques contenant des huiles éthyléniques, c'est-à-dire des copolymères éthyléniques, liquides à température ambiante, ayant une masse moléculaire moyenne en nombre comprise entre 150 et 5000.

L'emploi de cires présente certains inconvénients. En particulier, le taux de cristallinité de ces cires est difficilement contrôlable et les cristallites présents sont de grandes dimensions. Par suite, l'emploi de telles cires dans les compositions à application sur les matières kératiniques comme la peau, les lèvres et les phanères, en particulier cosmétiques, résulte en une matification des compositions et par suite du film appliqué.

Pour remédier à ce problème, on a proposé l'emploi de polyoléfines classiques à la place des cires. Mais là encore, le taux de cristallinité est trop élevé et difficilement contrôlable. En outre, la taille et la morphologie des cristallites, majoritairement de type sphérulite, nuisent à l'obtention de compositions ayant les propriétés cosmétiques souhaitées, en particulier de brillance.

La Société SHISEIDO a envisagé dans sa demande de brevet JP-A-65809 des compositions de rouge à lèvres contenant une résine siloxysilicate (à réseau tridimensionnel), une huile de silicone volatile à chaîne silicone cyclique et des charges pulvérulentes. De même, la Société NOEVIER a décrit dans le document JP-A-62-61911 des compositions de rouge à lèvres, d'eye-liner, de fonds de teint comportant une ou plusieurs cires hydrocarbonées. Egalement, la Société REVLON a décrit dans le document WO-A-97/17362 des compositions cosmétiques semi-mat, comprenant un solvant volatil et un émulsifiant polymère d'organosiloxane comportant au moins un radical ou une partie hydrophile et au moins un radical ou une partie lipophile.

Ces compositions ont l'inconvénient de laisser sur les lèvres, après évaporation des huiles de silicone, un film qui devient inconfortable au cours du temps (sensation de dessèchement et de tiraillement), écartant un certain nombre de femmes de ce type de rouge à lèvres.

La Société PROCTER & GAMBLE a envisagé dans sa demande de brevet WO-A-96/36323 des compositions de mascara de type émulsion eau-dans-huile présentant une bonne tenue et résistante à l'eau. Ces compositions contiennent, entre autre, un polymère insoluble dans l'eau, appelé généralement un latex, associé à un tensio-actif du type alkyle ou alcoxydiméthicone copolyol, des huiles hydrocarbonées, des pigments et charges, ainsi que des cires.

Dans le document WO-A-96/10642, la Société REVLON a décrit une composition cosmétique brillante, comprenant un polymère qui est un adhésif à température ambiante, un solvant volatil, une huile non volatile et une matière sèche particulaire. Les polymères adhésifs sont choisis parmi les polymères à squelette vinylique, méthacrylique ou acrylique et groupes pendants siloxane et fluorés, les polymères à squelette vinylique, méthacrylique ou acrylique et groupes pendants siloxanes, ainsi que les copolymères séquencés ou greffés vinyl-silicone.

En outre, les documents EP-A-497144 et FR-A-2 357 244 décrivent des compositions contenant un copolymère bloc styrène-éthylène-propylène associé à des cires, des huiles légères ou volatiles et des pigments. Ces compositions présentent l'inconvénient d'être peu confortables, d'avoir des propriétés cosmétiques quelconques et d'être difficilement formulables.

Il subsiste donc le besoin d'une composition ne présentant pas les inconvénients ci-dessus, et ayant un aspect plus ou moins brillant, adapté au désir de la consommatrice, ne desséchant pas au cours du temps la peau ou les lèvres sur lesquelles elle est appliquée et n'établissant pas d'inconfort.

La demanderesse a constaté, de façon tout à fait surprenante, que l'utilisation d'au moins un copolymère d'oléfines à cristallisation contrôlée et modérée, soluble ou dispersable dans une phase grasse, dans une composition cosmétique, dermatologique, pharmaceutique ou hygiénique et de façon plus générale physiologiquement acceptable, pouvait permettre d'obtenir un film de bonne tenue résistant à l'eau, tout en étant très agréable à l'application et à porter tout au long de la journée. Le film est notamment souple et flexible.

Les copolymères à cristallisation contrôlée et modérée, selon l'invention, présentent une taille et une morphologie spécifiques des cristallites. Ils comportent peu ou pas de cristallites de type sphérulite de grandes dimensions, mais au contraire principalement des cristallites de type lamellaire ou micelle frangée de petites dimensions. De préférence, les cristallites ont une dimension de 1 µm ou moins, mieux inférieure à 500 nm.

La présente invention a donc pour objet une composition à application sur les matières kératiniques, comprenant une phase grasse liquide, caractérisée par le fait qu'elle comprend une quantité efficace et en particulier au moins 2% en poids, par rapport au poids total de la composition, d'au moins un copolymère d'oléfines cristallin ayant une masse molaire moyenne en poids $\overline{M}_w \geq 30\ 000$ et un taux de cristallinité au plus égal à 50%, soluble ou dispersable dans la phase grasse liquide.

Cette composition est en particulier une composition cosmétique, dermatologique, hygiénique ou pharmaceutique. Elle contient donc des ingrédients compatibles avec la peau, les muqueuses et les phanères et plus spécialement les fibres kératiniques.

De préférence, cette composition contient en outre au moins une matière colorante.

Elle a également pour objet une composition se présentant sous forme d'un produit coulé et comprenant au moins une phase grasse liquide cosmétique, dermatologique, hygiénique ou pharmaceutique, et éventuellement au moins une cire solide à température ambiante, caractérisée par le fait qu'elle comprend, en outre, une quantité efficace et mieux au moins 2% en poids, par rapport au poids total de la composition, d'au moins un copolymère d'oléfines cristallin ayant une masse molaire moyenne en poids $\overline{M}_w \geq 30\ 000$ et un taux de cristallinité au plus égal à 50%, soluble ou dispersable dans la phase grasse liquide.

Comme précédemment, cette composition peut en outre contenir des ingrédients compatibles avec la peau, les muqueuses et les phanères et plus spécialement les fibres kératiniques, et en particulier au moins une matière colorante.

Le copolymère d'oléfines est présent en quantité efficace ou suffisante pour obtenir notamment un film de bonne tenue et/ou brillant et/ou résistant à l'eau.

Le ou les copolymères d'oléfines cristallins utilisés dans les compositions de la présente demande peuvent être tous copolymères d'oléfines, à savoir un copolymère comportant uniquement des motifs oléfiniques, ayant un caractère cristallin contrôlé et modéré, c'est-à-dire un taux de cristallinité au plus égal à 50%, de préférence compris entre 5 et 40%, et mieux compris entre 10 et 35%, bornes comprises.

Ces copolymères sont généralement des élastomères ou des plastomères et peuvent être synthétisés par tout procédé connu, en particulier par voie radicalaire, par catalyse Ziegler-Natta ou par catalyse métallocène, de préférence par catalyse métallocène. Les copolymères selon l'invention sont des solides (cires ou pâtes) à la température ambiante (25°C).

La copolymérisation peut être effectuée en masse, en solution ou en dispersion.

La cristallinité des copolymères de l'invention leur confère des propriétés épaississantes des huiles pouvant aller jusqu'à l'obtention d'un stick, des propriétés de transparence, un dépôt de film sur la peau ou les lèvres de façon aisée.

Les copolymères d'oléfines cristallins convenant dans la présente invention ont un point de fusion inférieur à 150°C, de préférence inférieur ou égal à 110°C.

Les copolymères d'oléfines cristallins selon l'invention ont de préférence une masse molaire moyenne en poids $\overline{M}_w \geq 40\ 000$ et un indice de polymolécularité $\leq 3,5$ (de préférence encore $\leq 2,5$), $\overline{M}_n$ étant la masse molaire moyenne en nombre.

Le taux de cristallinité des copolymères est déterminé comme cela est bien connu par calorimétrie différentielle à balayage (DSC) ou par diffraction des rayons-X pour les faibles taux de cristallinité.

Les copolymères d'oléfines préférés selon l'invention sont les copolymères d'oléfines obtenus par catalyse métallocène.

La catalyse métallocène permet un contrôle des propriétés du copolymère en ce qui concerne sa cristallinité, la longueur des chaînes polymères et l'homogénéité de la répartition des motifs dans les chaînes polymériques. Cette catalyse permet l'obtention de chaînes polymériques de même composition et à peu près de même longueur.

En effet, cette voie de synthèse permet un très bon contrôle des poids moléculaires des copolymères et conduit à une faible polydispersité (Indice de polymolécularité $\leq 2$). Elle permet un très bon contrôle de l'incorporation du comonomère dans les chaînes de polymères qui sont de composition chimique très voisine. De ce fait, on obtient un très bon contrôle de la cristallinité, c'est-à-dire du taux de cristallinité, de sa reproductibilité, et de la nature et dimension des cristallites formées.

Pour plus de détails quant aux avantages de cette synthèse par catalyse métallocène, on pourra se reporter aux articles "Emerging Technologies In Polymer Science and Engineering" (Technologies émergentes dans la Science et l'Ingenierie des Polymères) M.P. ZAMORA et al. - Plastics Engineering/May 97, pages 75 à 79 et "Classification of Homogeneous Ethylene-Octene Copolymers Based on Comonomer Content" (Classification des Copolymères Ethylène-Octène homogènes basée sur la teneur en Comonomère) S. BENSASON et al. - Journal of Polymer Science - Part B : Polymer Physics - Vol. 34, 130-135 (1996).

Dans les copolymères d'oléfines convenant dans la présente invention, la structure cristalline varie en fonction du taux de comonomère amorphe dans le copolymère.

Ainsi, dans le cas des copolymères éthylène/octène, comme le décrit l'article de S. BENSASON mentionné, lorsque la teneur en octène croît, on passe :

- pour une teneur en octène $\leq$ 2,5% en mole à des structures cristallines bien marquées avec présence de sphérulites, structures de type lamellaire appelées Type IV et les copolymères présentent alors des taux de cristallinité supérieurs à 50%;
- pour des teneurs en octène de l'ordre de 3% en mole à des structures encore fortement cristallines, lamellaires, mais avec des sphérulites plus petites (structure de type III), et les copolymères ont un taux de cristallinité de 38 à 50%;
- puis, pour des teneurs en octène de 5 à 6% en mole à des structures moins cristallines avec très peu de sphérulites et un mélange de structures en lamelles avec des "micelles frangées" (structure de type II) et les copolymères ont un taux de cristallinité de 28 à 38%;
- et, enfin, pour des teneurs en octène de 8 à 14% en mole à des structures encore plus faiblement cristallines ne comportant plus de sphérulites ni de lamelles, mais uniquement des "micelles frangées" (structure de type I), et les copolymères ont un taux de cristallinité de 10 à 28%.

Les copolymères recommandés pour la présente invention sont ceux ayant les structures de types I et II.

Les copolymères à structure de type IV, trop cristallins, ne conviennent pas pour la présente invention.

Le copolymère peut être choisi par l'homme du métier en fonction de ses propriétés et selon l'application ultérieure souhaitée pour la composition.

Ainsi, le polymère peut être filmifiable ou non.

L'invention a également pour objet une composition telle que définie ci-dessus, comprenant une phase grasse liquide volatile et comprenant au moins un actif choisi parmi les actifs cosmétiques, dermatologiques, hygiéniques ou pharmaceutiques.

Par "phase grasse volatile", on entend tout milieu non aqueux susceptible de s'évaporer de la peau ou des lèvres, en moins d'une heure.

La phase grasse volatile comporte notamment des huiles ou solvants ayant une pression de vapeur, à température ambiante (20-25°C) et pression atmosphérique, non nulle, allant de $10^{-3}$ à 300 mm de Hg. Par huile, on entend tout corps liquide non aqueux à température ambiante et pression atmosphérique.

Un autre objet de l'invention est l'utilisation dans ou pour la fabrication d'une composition sous forme de produit coulé et comprenant au moins une phase grasse liquide cosmétique, dermatologique, hygiénique ou pharmaceutique et au moins une cire, notamment solide à température ambiante, d'un copolymère d'oléfines cristallin ayant un taux de cristallinité au plus égal à 50%, soluble ou dispersable dans ladite phase grasse liquide, présent en une quantité efficace et notamment à au moins 2% en poids, par rapport au poids total de la composition, pour obtenir un film de bonne tenue et/ou brillant et/ou résistant à l'eau déposé sur les muqueuses comme les lèvres et/ou sur la peau.

Un autre objet de l'invention est l'utilisation dans ou pour la fabrication d'une composition à application sur les matières kératiniques, comprenant une phase grasse liquide et au moins un ingrédient choisi parmi les actifs cosmétiques, dermatologiques, hygiéniques et pharmaceutiques, les matières colorantes et leurs mélanges, d'au moins un copolymère d'oléfines cristallin ayant un taux de cristallinité au plus égal à 50%, soluble ou dispersable dans ladite phase grasse liquide, présent notamment en une quantité efficace, et en particulier en une quantité d'au moins 2% en poids, par rapport au poids total de la composition, pour obtenir un film de bonne tenue et/ou brillant et/ou résistant à l'eau déposé sur la peau et/ou les muqueuses comme les lèvres.

L'invention a encore pour objet un procédé de soin cosmétique ou de maquillage des lèvres ou de la peau, consistant à appliquer sur respectivement les lèvres ou la peau une composition cosmétique telle que définie précédemment.

Le copolymère peut être solubilisé dans la phase grasse de la composition par chauffage au dessus de son point de fusion.

De façon avantageuse, le copolymère se présente sous forme de particules dispersées et stabilisées en surface par au moins un stabilisant.

Un avantage de l'utilisation d'une dispersion de particules dans une composition de l'invention est que les particules restent à l'état de particules élémentaires, sans former d'agglomérats, dans la phase grasse, ce qui ne serait pas le cas avec des particules minérales de taille nanométrique. Un autre avantage de la dispersion de copolymère est la possibilité d'obtenir des compositions très fluides (de l'ordre de 130 centipoises), même en présence d'un taux élevé de copolymère.

On a de plus constaté que les compositions selon l'invention présentent des qualités d'étalement et d'adhésion sur la peau, les semi-muqueuses ou les muqueuses, particulièrement intéressantes, ainsi qu'un toucher onctueux et agréable. Ces compositions ont, en outre, l'avantage de se démaquiller facilement, notamment avec un lait démaquillant classique.

Une première classe de copolymères d'oléfines cristallins, utilisables dans les compositions selon l'invention, comprend les copolymères d'$\alpha$-oléfine, en particulier d'$\alpha$-oléfine en $C_2$-$C_{16}$ et mieux en $C_2$-$C_{12}$. Ces copolymères ont en général une densité (d) à température ambiante (20-25°C) telle que $0,86 \leq d \leq 0,91$ et, de préférence $0,86 \leq d \leq 0,905$. De préférence, ces copolymères sont des bi- ou terpolymères et tout particulièrement des bipolymères.

Parmi les bipolymères recommandés pour les compositions de l'invention, on peut citer les bipolymères d'éthylène et d'$\alpha$-oléfine en $C_4$-$C_{16}$, de préférence en $C_4$-$C_{12}$ et les bipolymères de propylène et d'$\alpha$-oléfine en $C_4$-$C_{16}$, de préférence en $C_4$-$C_{12}$. De préférence encore, l'$\alpha$-oléfine est choisie parmi le butène-1, le pentène-1, l'hexène-1, l'heptène-1, l'octène-1, le nonène-1, le décène-1, l'undécène-1, le dodécène-1, le 3,5,5-triméthylhexène-1, le 3-méthylpentène-1, et le 4-méthylpentène-1.

Parmi ces monomères, le butène-1 et l'octène-1 sont particulièrement préférés.

Le taux d'$\alpha$-oléfine dans le bipolymère est généralement compris entre 2 et 40% en mole, de préférence 3 à 30% en mole, et mieux 4 à 20% en mole.

Les bipolymères éthylène-octène recommandés sont les plastomères ayant une teneur en octène comprise entre 5,2% et 6,2% en mole, un taux de cristallinité compris entre 28 et 38% et les élastomères ayant une teneur en octène entre 8 et 14% en mole et un taux de cristallinité compris entre 10 et 28%.

Ces bipolymères sont synthétisés par catalyse métallocène.

De tels bipolymères sont commercialisés par la Société DOW CHEMICAL sous les dénominations commerciales AFFINITY® (plastomères) et ENGAGE® (élastomères).

Des bipolymères éthylène-butène sont commercialisés par la Société EXXON sous l'appellation commerciale EXACT RESINS®.

Parmi les terpolymères, on peut citer les terpolymères d'éthylène, de propylène et d'$\alpha$-oléfine en $C_4$-$C_{16}$, de préférence $C_4$-$C_{12}$.

Dans ces terpolymères, les teneurs en $\alpha$-oléfine en $C_4$-$C_{16}$ sont comme indiquées précédemment et les $\alpha$-oléfines préférées sont le butène, l'hexène et l'octène.

Parmi les copolymères d'$\alpha$-oléfines convenant dans la présente invention, on peut également citer les copolymères décrits dans le document EP-81787.

Une seconde classe de copolymères d'oléfines convenant pour les compositions selon l'invention, comprend les copolymères d'éthylène et/ou de propylène et d'une cycloolefine, en particulier les bipolymères.

Généralement, la teneur en cycloolefine des copolymères est inférieure à 20% en mole.

Parmi les cycloolefines utilisables, on peut citer le cyclobutène, le cyclohexène, le cyclooctadiène, le norbornène, le diméthano-octahydronaphtalène (DMON), l'éthylidène norbornène, le vinyl norbornène et le 4-vinylcyclohexène.

Les copolymères recommandés de cette classe sont les copolymères d'éthylène et de norbornène. La teneur en norbornène de ces copolymères est généralement inférieure à 18% en mole pour présenter le caractère cristallin requis et ces copolymères sont synthétisés par catalyse métallocène.

Des copolymères éthylène/norbornène appropriés sont commercialisés par les Sociétés MITSUI PETROCHEMICAL ou MITSUI-SEKKA sous la dénomination commerciale APEL® et par la Société HOECHST-CELANESE sous la dénomination commerciale TOPAS®.

D'autres copolymères d'éthylène/cycloolefine recommandés sont les bipolymères éthylène/cyclobutène et éthylène/cyclohexène à faible teneur en cycloolefine, généralement inférieure à 20% en mole.

Une troisième classe de copolymères d'oléfines appropriés est constituée par les copolymères d'oléfines de tacticité contrôlée, c'est-à-dire des copolymères comportant des motifs de tacticité différente.

Parmi ces copolymères de tacticité contrôlée, on peut citer les copolymères propylène isotactique/propylène atactique et propylène syndiotactique/propylène atactique.

Les motifs ou séquences iso- ou syndiotactiques confèrent au copolymère le caractère cristallin, cependant que les motifs ou séquences atactiques amorphes empêchent une trop forte cristallinité du copolymère et règlent le taux de cristallinité ainsi que la morphologie et la taille des cristallites.

La teneur en motifs iso- ou syndiotactiques, motifs conférant le caractère cristallin au copolymère, est donc déterminée pour obtenir le pourcentage de cristallinité voulu ($\leq 50\%$) dans le copolymère.

La teneur en motifs tactiques est généralement comprise entre 10 et 80% en mole. Toutefois, de préférence, la teneur en motifs tactiques est inférieure à 30% en mole.

Ces copolymères sont synthétisés par catalyse métallocène.

Une quatrième classe de copolymères d'oléfines convenant pour la présente invention, est constituée par les copolymères de monooléfines et de monomères à liaison(s) éthylénique(s), différents des monooléfines, tels que les diènes, par exemple les bipolymères éthylène/butadiène, propylène/butadiène, éthylène/isoprène et propylène/isoprène, et les terpolymères éthylène/propylène/diène, obtenus également par synthèse métallocène.

La proportion de motifs monomères à liaison (s) éthylénique(s) tels que les diènes dans le copolymère à cristallisation contrôlée est généralement comprise entre 3 et 20% en mole.

Pour améliorer le réglage de la cristallinité du copolymère, on peut éventuellement ajouter à la composition selon l'invention des additifs gênant la cristallisation et favorisant la formation de petits cristaux. Ces additifs, bien qu'utilisés en faible proportion, constituent des "sites" de germination nombreux et petits répartis uniformément dans la masse. Ces additifs sont typiquement des cristaux d'une substance organique ou minérale.

Dans le cas d'un additif organique devant cristalliser, celui-ci doit avoir un point de fusion supérieur à la zone de fusion du copolymère et former, de préférence, de petits cristaux.

A une température supérieure à son point de fusion, cette substance est de préférence soluble dans le mélange de la phase grasse liquide et de polymère fondu. Ainsi, lors du refroidissement, l'additif initialement dissous, recristallise sous forme de petits cristaux nombreux et bien diffusés dans le mélange, puis le polymère recristallise en donnant des domaines cristallins petits du fait de la présence des cristaux d'additifs. Cette technique de recristallisation des polymères est classique.

On peut également ajuster le taux de cristallisation, la taille et la morphologie des copolymères d'oléfines selon l'invention en mélangeant un premier copolymère d'oléfines selon l'invention avec un second polymère ou copolymère cristallin, compatible en partie avec le premier copolymère d'oléfines. Le second polymère ou copolymère peut être un copolymère d'oléfines selon l'invention, mais de taux de cristallinité différent de celui du premier copolymère, y compris un taux de cristallinité plus élevé que le taux de cristallinité des copolymères d'oléfines selon l'invention.

Le deuxième polymère cristallisable peut aussi être un polymère de nature différente, par exemple un copolyéthylène/acétate de vinyle obtenu par copolymérisation radicalaire ou même un polyéthylène cristallisable tel que ceux habituellement utilisés dans le domaine cosmétique.

Pour plus de détails quant à cette méthode d'ajustement du taux de cristallinité, on se référera aux articles intitulés "Elastomeric blends of homogeneous ethylene-octene copolymers (Mélanges élastomériques de copolymères homogènes éthylène-octène)" S. Bensason et al., Polymer, Volume 38, N° 15, 1997, pages 3913-19, et "Blends of homogeneous ethylene-octene copolymers (Mélanges de copolymères homogènes éthylène-octène)" S; Bensason et al., Polymer, Volume 38, N° 14, 1997, pages 3513-20.

La phase grasse liquide dans laquelle est dispersé le copolymère, peut être constituée de toute huile cosmétiquement ou dermatologiquement acceptable, et de façon générale physiologiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

Par "phase grasse liquide", on entend tout milieu non aqueux liquide à température ambiante, 20-25°C et pression atmosphérique.

On peut ainsi citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales; des esters d'acide lanolique, d'acide oléique, d'acide laurique, d'acide stéarique; les esters gras, tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine; les acides gras supérieurs à au moins 12 atomes de carbone tels que l'acide myristique, l'acide palmitique, l'acide stéarique, l'acide béhénique, l'acide oléique, l'acide linoléique, l'acide linolénique ou l'acide isostéarique; les alcools gras supérieurs à au moins 12 atomes de carbone tels que l'alcool stéarylique ou l'alcool oléique, l'alcool linoléique ou linolénique, l'alcool isostéarique ou l'octyl dodécanol; les huiles siliconées telles que les polydiméthylsiloxanes (PDMS), éventuellement phénylées telles que les phényltriméthicones, les phényltriméthylsiloxydiphénylsiloxanes, les diphénylméthyldiméthyltrisiloxanes, les diphényldiméthicones, les phényldiméthicones, les polyméthylphénylsiloxanes, ou éventuellement substitués par des groupements aliphatiques et/ou aromatiques, éventuellement fluorés, ou par des groupements fonctionnels tels que des groupements hydroxyle, thiol et/ou amine; les polysiloxanes modifiés par des acides gras, des alcools gras ou des polyoxyalkylènes, les silicones fluorées, les huiles perfluorées.

Parmi les huiles siliconées préférées, on peut citer les polydiméthylsiloxanes, les polyméthylphénylsiloxanes, les silicones comportant des séquences ou des greffons polyoxyalkylènes, en particulier polyoxyéthylène ou copoly(oxyéthylène-oxypropylène) telles que les diméthiconecopolyols, les silicones portant à la fois des groupes hydrophobes hydrocarbonés (par exemple des groupes alkyle en $C_2$-$C_{30}$) et des séquences ou greffons polyoxyéthylénés ou copoly(oxyéthylénés/ oxypropylénés) telles que les alkyldiméthiconecopolyols, les silicones portant des groupes fluorés ou perfluorés telles que les polydiméthyl siloxanes perfluoroalkylés et les polyméthylphénylsiloxanes perfluoro alkylés.

Avantageusement, on peut utiliser une ou plusieurs huiles volatiles à température ambiante. Après évaporation de ces huiles, on obtient un dépôt filmogène souple. Ces huiles volatiles facilitent, en outre, l'application de la composition sur la peau, les muqueuses, les phanères.

Ces huiles peuvent être des huiles hydrocarbonées, des huiles silicones comportant éventuellement des groupements alkyle ou alcoxy en bout de chaîne siliconée ou pendante.

Comme huile de silicone volatile utilisable dans l'invention, on peut citer les silicones linéaires ou cycliques ayant de 2 à 7 atomes de silicium, ces silicones comportant éventuellement des groupes alkyle ou alcoxy ayant de 1 à 10 atomes de carbone ainsi que les isoparaffines en $C_8$-$C_{16}$ et les huiles fluorées ou perfluorées volatiles. Ces huiles volatiles représentent notamment de 30 à 97,99% du poids total de la composition, et mieux de 30 à 75%.

Comme huile volatile utilisable dans l'invention, on peut citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane ou les isoparaffines en $C_8$-$C_{16}$ telles que les 'ISOPARs', les PERMETHYLs et notamment l'isododécane ou l'isohexadécane.

Dans un mode particulier de réalisation de l'invention, on choisit la phase grasse liquide dans le groupe comprenant :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges.

Le paramètre de solubilité global $\delta$ selon l'espace de solubilité de HANSEN est défini dans l'article "Solubility parameter values" de Eric A. Grulke, de l'ouvrage "Polymer Handbook", 3ème édition, Chapitre VII, pages 519-559, par la relation

$$\delta = (d_p^{\ 2} + d_p^{\ 2} + d_H^{\ 2})^{1/2}$$

dans laquelle

- $d_p$ caractérise les forces de dispersion de LONDON issues de la formation de dipôles induits lors des chocs moléculaires,
- $d_p$ caractérise les forces d'interactions de DEBYE entre dipôles permanents,

- $d_H$ caractérise les forces d'interactions spécifiques (type liaisons hydrogène, acide/base, donneur/accepteur, etc.). La définition des solvants dans l'espace de solubilité tridimensionnel selon HANSEN, est décrite dans l'article de C.M. HANSEN : "The three dimensional solubility parameters", J. Paint Technol. 39, 105 (1967).

Parmi les phases grasses liquides ayant un paramètre de solubilité global selon l'espace de solubilité de HANSEN inférieur ou égal à 17 $(MPa)^{1/2}$, on peut citer des huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR', dans laquelle R représente le reste d'un acide gras supérieur comportant de 7 à 19 atomes de carbone, et R' représente une chaîne hydrocarbonée comportant de 3 à 20 atomes de carbone, tels que les palmitates, les adipates et les benzoates, notamment l'adipate de diisopropyle.

On peut également citer les hydrocarbures et notamment des huiles de paraffine, de vaseline, ou le polyisobutylène hydrogéné, l'isododécane, ou encore les 'ISOPARs', isoparaffines volatiles.

On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyle, thiol et/ou amine, et les huiles siliconées volatiles, notamment cycliques.

On peut également citer les solvants, seuls ou en mélange, choisis parmi :

(i) les esters linéaires, ramifiés ou cycliques, ayant plus de 6 atomes de carbone,
(ii) les éthers ayant plus de 6 atomes de carbone,
(iii) les cétones ayant plus de 6 atomes de carbone.

Par monoalcools ayant un paramètre de solubilité global selon l'espace de solubilité de HANSEN inférieur ou égal à 20 $(MPa)^{1/2}$, on entend les alcools gras aliphatiques ayant au moins 6 atomes de carbone, la chaîne hydrocarbonée ne comportant pas de groupement de substitution.

Comme monoalcools selon l'invention, on peut citer l'alcool oléique, le décanol, de dodécanol, l'octadécanol et l'alcool linoléique.

Comme milieu non aqueux, on peut aussi utiliser ceux décrits dans le document FR-A-2 710 646 de L.V.M.H.

Le choix du milieu non aqueux est effectué par l'homme du métier en fonction de la nature des monomères constituant le copolymère.

De plus, la phase grasse liquide dans laquelle est dissous ou dispersé le copolymère peut représenter de 30% à 97,99% du poids total de la composition, et de préférence de 30 à 75%.

La composition peut comprendre une matière colorante contenant un ou plusieurs composés pulvérulents et/ou un ou plusieurs colorants liposolubles, par exemple à raison de 0,01 à 70% du poids total de la composition. Les composés pulvérulents peuvent être choisis parmi les pigments et/ou les nacres et/ou les charges habituellement utilisés dans les compositions cosmétiques ou dermatologiques. Les composés pulvérulents peuvent représenter de 0,1 à 98% du poids total de la composition, et par exemple de 1 à 80%. Plus la quantité de composés pulvérulents diminue, plus les qualités de confort augmentent. Aussi, de préférence ces composés pulvérulents représentent de 0,1 à 40%, et mieux de 1 à 30%.

En pratique, le copolymère peut représenter jusqu'à 60% (en matière active ou sèche) du poids total de la composition, de préférence 12 à 60% en poids.

De façon préférentielle, le rapport en poids de pigment(s)/copolymère est < 1 et même ≤ 0,9. De préférence, ce rapport est ≤ 0,5. Ce rapport peut descendre jusqu'à 0,015.

La composition de l'invention peut comprendre, avantageusement, au moins 30% en poids de phase grasse, par rapport au poids total de la composition. En dessous de 30%, on obtient une texture granuleuse et pulvérulente. Ceci est peu souhaitable lorsque l'on cherche à obtenir un aspect crémeux, gélifié ou en stick, homogène non granuleux.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert d'oxyde de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon (Orgasol de chez Atochem), de poly-β-alamine et de polyéthylène, le Téflon, la lauroyl-lysine, l'amidon, le nitrure de bore, les poudres de polymères de tétrafluoroéthylène, les microsphères creuses telles l'Expancel (Nobel Industrie), le polytrap (Dow Corning) et les microbilles de résine de silicone (Tospearls de Toshiba, par exemple), le carbonate de calcium précité, la carbonate et l'hydro-carbonate de magnésium, l'hydroxyapatite, les microsphères de silice creuses (SILICA BEADS DE MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les charges utilisées, en particulier les charges organiques de nature polymère, peuvent être réticulées ou non et contenir à l'intérieur des particules un ou plusieurs actifs cosmétiques, dermatologiques, hygiéniques ou pharmaceutiques pouvant être libérés après application de la composition.

Les pigments et les charges peuvent être ou non enrobés superficiellement, en particulier traités en surface par des silicones, des acides aminés, des dérivés fluorés ou toute autre substance favorisant la dispersion et la compatibilité du pigment dans la composition.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Ils peuvent représenter de 0,01 à 20 % du poids de la composition et mieux de 0,1 à 6 %.

Le copolymère de la composition de l'invention permet la formation d'un film sur la peau, les lèvres et/ou les muqueuses, formant un réseau piégeant les matières colorantes et/ou les actifs. Selon la quantité relative de matières colorantes, utilisées par rapport à la quantité de polymère stabilisé, utilisée, il est possible d'obtenir un film plus ou moins brillant.

Comme actifs cosmétiques, dermatologiques, hygiéniques ou pharmaceutiques utilisables dans la composition de l'invention, on peut citer les huiles cosmétiques, les hydratants, vitamines, acides gras essentiels, sphingolipides, filtres solaires, les antioxydants, les antiacnés, les anti-inflammatoires, les agents bronzants (en l'absence de rayonnement UV), les agents dépigmentants, les agents mâtifiants et leurs mélanges. Parmi les agents hydratants, on peut citer le glycérol et le polyméthacrylate de glycérol, le hyaluronate de sodium et les esters de polyol ou de sucres. Ces actifs sont utilisés en quantité habituelle pour l'homme du métier et notamment à des concentrations de 0,001 à 20 % du poids total de la composition.

La composition selon l'invention peut, de plus, comprendre, selon le type d'application envisagée, les constituants classiquement utilisés dans les domaines considérés, qui sont présents en une quantité appropriée à la forme galénique souhaitée.

En particulier, elle peut comprendre, outre, la phase grasse liquide dans laquelle le polymère est stabilisé, des phases grasses additionnelles qui peuvent être choisies parmi les cires, les huiles, les gommes et/ou les corps gras pâteux, d'origine végétale, animale, minérale ou de synthèse, voire siliconé, et leurs mélanges.

Parmi les cires solides à température ambiante, susceptibles d'être présentes dans la composition selon l'invention, on peut citer les cires ayant en particulier un point de fusion supérieur à 45°C comme les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25°C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Microemulsions Theory and Practice, L. M. Prince Ed., Academic press (1977, pages 21-32)". Comme cire liquide à température ambiante, on peut citer l'huile de Jojoba.

Les cires peuvent être présentes à raison de 0-50 % en poids dans la composition et mieux de 10 à 30 %.

La composition peut comprendre, en outre, tout additif usuellement utilisé dans de telles compositions, tel que des épaississants, des parfums, des conservateurs, des tensioactifs, des polymères liposolubles comme les polyalkylènes, notamment le polybutène, les polyacryliques et les polymères siliconés compatibles avec la phase grasse ainsi que les dérivés de polyvinylpyrrolidone. Bien entendu l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Parmi les épaississants, on peut citer les bentonites, les silices traitées, les gommes de guar alkylées liposolubles, les polymères séquencés ou greffés comportant au moins une séquence soluble dans la composition et une séquence insoluble tel que, par exemple, les copolymères bi ou triséquencés polystyrène/copoly(éthylène-propylène) ou polystyrène/copoly(éthylène-butylène), les (polyvinyl)pyrrolidone hexadiène, les gommes de silicone et les silicones KSG.

Les gommes de silicone ont en général une masse molaire moyenne en nombre comprise entre 200.000 et 1.000.000. A titre d'exemple de gommes de silicone qui peuvent être utilisées seules ou sous forme de mélange dans un solvant, on peut citer les copolymères suivants :

- poly[(diméthylsiloxane)/(méthylvinylsiloxane)]
- poly[(diméthylsiloxane)/(diphénylsiloxane)]
- poly[(diméthylsiloxane)/(phénylméthylsiloxane)]
- poly[(diméthylsiloxane)/(diphénylsiloxane)/(méthylvinylsiloxane)] ; et les mélanges suivants :

- les mélanges formés à partir d'un polydiméthylsiloxane hydroxylé en bout de chaîne et d'un polydiméthylsiloxane cyclique;
- les mélanges formés à partir d'une gomme de polydiméthyl siloxane et d'une silicone cyclique; et
- les mélanges de deux polydiméthylsiloxanes de viscosités différentes.

Les compositions selon l'invention peuvent en outre contenir des homopolymères et copolymères liposolubles et/ou dispersables dans la phase grasse, différents des copolymères d'oléfines à cristallisation contrôlée selon l'invention.

Parmi ces homopolymères et copolymères, on peut citer des polyoléfines telles que le polyéthylène, le polybutène et le polydécène; des copolymères d'esters et/ou amides (méth)acryliques; des copolymères d'esters vinyliques, par exemple des copolymères éthylène/acétate de vinyle; des homopolymères ou copolymères vinyliques ou (méth)acryliques portant un groupement silicone tels que par exemple des copolymères greffés à squelette (méth)acrylique et greffons de silicone macromère; des copolymères à squelette ou séquences (méth)acrylique et à greffons ou séquences hydrocarbonés, par exemple polyisobutylène; des copolymères greffés ou séquencés à squelette ou séquence polyorganosiloxane et à greffons ou séquences (méth)acryliques et/ou vinyliques; des homopolymères ou copolymères fluorés ou perfluorés, par exemple des polyéthers perfluorés tels ceux commercialisés sous la désignation FOMBLINS®, des homo ou copolymères (méth)acryliques perfluorés, des homo ou copolymères vinyliques fluorés, des homo ou copolymères d'oléfines fluorés et des poly(éther vinyliques) fluorés.

Dans un mode de réalisation particulier de l'invention, les compositions selon l'invention peuvent être préparées de manière usuelle par l'homme du métier. Elle peuvent se présenter sous forme d'un produit coulé et par exemple sous la forme d'un stick ou bâton, ou sous la forme de coupelle utilisable par contact direct ou l'éponge. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple, de viscosité dynamique à 25°C de l'ordre de 1 à 40 Pa.s ou encore de gel, de crème plus ou moins fluide. Elles peuvent alors constituer des fonds de teint ou des rouges à lèvres, des produits solaires ou de coloration de la peau.

De préférence, les compositions selon l'invention ont une viscosité dynamique n ≥ 5 Pa.s telle que mesurée à 25°C avec un rhéomètre à contrainte imposée HAAKE RS 75 en géométrie cône-plan (caractéristique du cône, 20 mm de diamètre, 1° d'angle et 40 μm de "gap").

Les compositions de l'invention sont avantageusement anhydres et peuvent contenir moins de 5% d'eau par rapport au poids total de la composition. Elles peuvent alors se présenter notamment sous forme de gel huileux, de liquide huileux ou huile, de pâte ou de stick. Ces formes galéniques sont préparées selon les méthodes usuelles des domaines considérés.

Les compositions selon l'invention peuvent également avantageusement se présenter sous forme d'émulsion eau-dans-huile, huile-dans-eau ou eau-dans-cire, dans lesquelles les copolymères d'oléfines selon l'invention sont utilisés pour remplacer tout ou partie des cires habituellement présentes dans ces émulsions. En particulier, la cire peut être constituée d'au moins un copolymère d'oléfines selon l'invention et d'au moins une huile, volatile ou non.

Les compositions peuvent être sous forme de dispersions vésiculaires contenant des lipides ioniques et/ou non ioniques.

Ces compositions à applications topique peuvent constituer notamment une composition cosmétique, dermatologique, hygiénique ou pharmaceutique de protection, de traitement ou de soin pour le visage, pour le cou, pour les mains ou pour le corps (par exemple crème de soin anhydre, huile solaire, gel corporel), une composition de maquillage (par exemple gel de maquillage) ou une composition de bronzage artificiel.

Les exemples suivants, non limitatifs, illustrent la présente invention.

**Exemple 1** : préparation d'un stick rouge à lèvres.

Choix du polymère

Copolymère (éthylène/1-octène) avec 4,7% en mole d'octène, préparé par synthèse métallocène, et vendu par DOW CHEMICAL sous le nom d'ENGAGE® CGCT 8817-O (taux de cristallinité : 25%) et avec les caractéristiques données dans l'article de J. MINICK, J. of Appl. Polym. Sci, Vol. 58, 1371-84 (1995).

Composition de la formule :

- Polymère        14 g
- Lanoline        7 g
- Capric/caprylique triglycéride        13 g
- Huile de sésame        22 g
- Cyclopentadiméthylsiloxane        32 g
- Pigment (oxydes de fer)        12 g

On mélange à chaud tous les constituants, sauf la silicone volatile, en chauffant à 105°C pour dissoudre le polymère. Après homogénéisation et broyage des pigments, on rajoute alors la silicone volatile à 90°C et on coule le mélange dans un moule adéquat.

On obtient un stick de bonnes caractéristiques rhéologiques qui dépose après application sur les lèvres un film confortable.

**Exemple 2** : préparation d'un rouge à lèvres.

Choix du polymère :

Copolymère (éthylène/1-octène) préparé par synthèse métallocène, et vendu par DOW CHEMICAL sous le nom d'ENGAGE® 8400 (taux de cristallinité 10-35%).

Composition de la formule :

- Polymère ENGAGE® 8400        18 g
- Pigment (oxydes de fer)        6 g
- Isododécane        50 g
- Huile de Parleam        26 g

On mélange à chaud tous les constituants et on coule le mélange dans un moule adéquat.

On obtient un stick de bonnes caractéristiques rhéologiques qui dépose après application sur les lèvres un film confortable.

**Revendications**

1. Composition à application sur les matières kératiniques, comprenant une phase grasse liquide et une quantité efficace d'au moins un copolymère soluble ou dispersable dans la phase grasse liquide, **caractérisée par le fait que** le copolymère a une masse molaire moyenne en poids $\overline{M}_w$ telle que $M_w \geq 30\,000$, et est choisi parmi les copolymères d'oléfines cristallins ayant un taux de cristallinité au plus égal à 50 %, de préférence compris entre 5 et 40%, et mieux compris entre 10 et 35%, bornes comprises.

2. Composition selon la revendication 1, **caractérisée en ce que** le copolymère représente au moins 2% en poids, par rapport au poids total de la composition.

3. Composition selon la revendication 1 ou 2, **caractérisée en ce que** le copolymère a une masse molaire moyenne en poids $\overline{M}_w$ telle que $\overline{M}_w \geq 40\,000$.

4. Composition selon la revendication 3, **caractérisée en ce que** le copolymère a un indice de polymolécularité $\dfrac{\overline{M}_w}{\overline{M}_n} \leq 3{,}5$, de préférence $\leq 2{,}5$, où $\overline{M}_n$ est la masse molaire moyenne en nombre.

5. Composition selon l'une quelconque des revendications précédentes, **caractérisée en ce que** le copolymère est un copolymère solide à la température ambiante.

**6.** Composition selon l'une quelconque des revendications précédentes, **caractérisée par le fait qu'**elle se présente sous la forme d'un produit coulé comprenant une phase grasse liquide cosmétique, dermatologique, hygiénique ou pharmaceutique et au moins une cire solide.

**7.** Composition selon l'une des revendications précédentes, **caractérisée par le fait que** le copolymère est filmifiable.

**8.** Composition selon la revendication 7, **caractérisée par le fait que** la phase grasse liquide est volatile.

**9.** Composition selon la revendication 8, **caractérisée par le fait qu'**elle comprend, en outre, au moins un actif choisi parmi les actifs cosmétiques, dermatologiques, hygiéniques ou pharmaceutiques et leurs mélanges.

**10.** Composition selon l'une quelconque des revendications précédentes, **caractérisée par le fait qu'**elle comprend, de plus, au moins une matière colorante.

**11.** Composition selon l'une quelconque des revendications précédentes, **caractérisée par le fait que** le copolymère d'oléfines est choisi parmi :

(A) les copolymères d'$\alpha$-oléfines, les copolymères d'oléfines et de cyclooléfines, les copolymères d' $\alpha$-oléfines et de monomères à liaison(s) éthylénique(s) tels que les diènes ; et
(B) les copolymères d' $\alpha$-oléfines à motifs tactiques et atactiques.

**12.** Composition selon la revendication 11, **caractérisée par le fait que** les copolymères d' $\alpha$-oléfines sont choisis parmi les bipolymères d'éthylène ou de propylène et d' $\alpha$-oléfine en $C_4$-$C_{16}$, de préférence $C_4$-$C_{12}$, et les terpolymères d'éthylène, de propylène et d' $\alpha$-oléfine en $C_4$-$C_{16}$, de préférence $C_4$-$C_{12}$.

**13.** Composition selon la revendication 12, **caractérisée par le fait que** le l' $\alpha$-oléfine en $C_4$-$C_{16}$ est choisie parmi le butène-1, le pentène-1, l'hexène-1, l'heptène-1, l'octène-1, le nonène-1, le décène-1, l'undécène-1, le dodécène-1, le 3,3,5-triméthylhexène-1, le 3-méthyl pentène-1, et le 4-méthylpentène-1.

**14.** Composition selon la revendication 12 ou 13, **caractérisée par le fait que** le pourcentage en mole d'$\alpha$-oléfine est au plus égal à 40%, de préférence égal ou inférieur à 30%.

**15.** Composition selon la revendication 11, **caractérisée par le fait que** les copolymères d'oléfines sont choisis parmi les bipolymères d'éthylène ou de propylène avec le cyclobutène, le cyclohexène, le cyclo octadiène, le norbornène, le diméthano-octahydronaphtalène (DMON), l'éthylidènenorbornène, le vinylnorbornène et le 4-vinylcyclohexène et les terpolymères d'éthylène, de propylène et des cyclooléfines précédentes.

**16.** Composition selon la revendication 15, **caractérisée par le fait que** le copolymère d'oléfine et de cyclooléfine contient moins de 20% en mole de cyclooléfine.

**17.** Composition selon la revendication 16, **caractérisée par le fait que** le copolymère d'$\alpha$-oléfine et de cyclooléfine est un copolymère éthylène/norbornène contenant moins de 18% en mole de norbornène.

**18.** Composition selon la revendication 11, **caractérisée par le fait que** les copolymères d'$\alpha$-oléfine et de monomères à liaison(s) éthylénique(s) tels que les diènes sont choisis parmi les bipolymères éthylène/butadiène et éthylène/isoprène.

**19.** Composition selon la revendication 18, **caractérisée par le fait que** le bipolymère contient moins de 20% en mole de monomères à liaison(s) éthylénique(s).

**20.** Composition selon la revendication 11, **caractérisée par le fait que** les copolymères d'$\alpha$-oléfines à motifs tactiques et atactiques sont choisis parmi les polypropylènes à motifs isotactiques et atactiques et les polypropylènes à motifs syndiotactiques et atactiques.

**21.** Composition selon la revendication 20, **caractérisée par le fait que** le taux de motifs tactiques est inférieur à 30% en mole.

**22.** Composition selon l'une quelconque des revendications précédentes, **caractérisée par le fait que** les copolymères d'oléfines sont obtenus par synthèse métallocène.

**23.** Composition selon l'une des revendications précédentes, dans laquelle la phase grasse liquide est constituée d'huiles d'origine minérale, animale, végétale ou synthétique, carbonées, hydrocarbonées, fluorées et/ou siliconées, seules ou en mélange.

**24.** Composition selon l'une des revendications précédentes, dans laquelle la phase grasse liquide est choisie parmi l'huile de paraffine ou de vaseline, l'huile de vison, de tortue, de soja, le perhydrosqualène, l'huile d'amande douce, de calophyllum, de palme, de parléam, de pépins de raisin, de sésame, de maïs, de colza, de tournesol, de coton, d'abricot, de ricin d'avocat, de jojoba, d'olive ou de germes de céréales ; des esters d'acide lanolique, d'acide oléique, d'acide laurique, d'acide stéarique ; les esters gras tels quele myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyldodécyle, le succinate de 2-diéthyl-hexyle, le malate de diisostéaryle, le triisostéarate de glycérineou de diglycérine ; les acides gras supérieurs à au moins 12 atomes de carbone tels que l'acide myristique, l'acide palmitique, l'acide stéarique, l'acide béhénique, l'acide oléique, l'acide linoléique, l'acide linolénique ou l'acide isostéarique les alcools gras supérieurs à au moins 12 atomes de carbone tels que l'alcool stéarylique ou l'alcool oléique, l'alcool linoléique ou linolénique, l'alcool isostéarique ou l'octyl dodécanol ; les huiles siliconées telles que les PDMS éventuellement phénylés tels que les phényltriméthicones, les phényltriméthylsiloxydiphénylsiloxanes, les diphénylméthyldiméthyltrisiloxanes, les diphényldiméthicones, les phényldiméthicones, les polyméthylphénylsiloxanes ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées; les huiles volatiles telles que l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane ou les isoparaffines en $C_8$-$C_{16}$, et notamment l'isododécane et l'isohexadécane.

25. Composition selon l'une des revendications précédentes, dans laquelle la phase grasse liquide est choisie dans le groupement comprenant :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges.

26. Composition selon l'une quelconques des revendications précédentes, dans laquelle la phase contient au moins une huile volatile à température ambiante.

27. Composition selon l'une des revendications précédentes, comprenant, en outre, au moins une phase grasse additionnelle choisie parmi les cires, les gommes et/ou les corps gras pâteux, d'origine végétale, animale, minérale, de synthèse, ou siliconé, et leurs mélanges.

28. Composition selon l'une quelconque des revendications précédentes, **caractérisée en ce qu'**elle comprend au moins un composé pulvérulent choisi parmi les charges, les pigments, les nacres et leurs mélanges.

29. Composition selon la revendication 28, **caractérisée en ce que** le composé pulvérulent et le copolymère sont présents dans un rapport pigments/copolymère inférieur à 1.

30. Composition selon la revendication 28 ou 29, **caractérisée en ce que** le composé pulvérulent représente 0,1 à 98% du poids total de la composition.

31. Composition selon la revendication 29, **caractérisée en ce que** le composé pulvérulent représente de 1 à 30% du poids total de la composition.

32. Composition selon l'une des revendications précédentes, **caractérisée en ce que** le copolymère d'oléfines représente (en matière sèche) jusqu'à 60% du poids total de la composition.

33. Composition selon l'une des revendications précédentes, **caractérisée en ce que** le copolymère d'oléfines représente (en matière sèche) de 12 à 60% du poids total de la composition.

34. Composition selon l'une des revendications précédentes, **caractérisée en ce que** la phase grasse liquide contient au moins une huile choisie parmi les isoparaffines en $C_8$-$C_{16}$ et les silicones linéaires ou cycliques ayant de 2 à 7 atomes de silicium, ces silicones comportant éventuellement des groupes alkyles ayant de 1 à 10 atomes de carbone, et leurs mélanges.

35. Composition selon l'une des revendications précédentes, se présentant sous forme d'un stick ou bâton, sous la forme d'une pâte souple, de viscosité dynamique à 25°C de l'ordre de 1 à 40 Pa.s ; sous forme de coupelle ; de gel huileux ; de liquide huileux ; de dispersion vésiculaire contenant des lipides ionique et/ou non ionique.

36. Composition selon l'une quelconque des revendications 1 à 35, **caractérisée en ce qu'**elle se présente sous forme d'une émulsion eau-dans-huile, huile-dans-eau ou eau-dans-cire, ladite cire pouvant être constituée par un mélange d'au moins un copolymère d'oléfines cristallin ayant un taux de cristallinité au plus égal à 50% et au moins une huile.

**37.** Composition selon l'une quelconque des revendications 1 à 34, se présentant sous forme anhydre.

**38.** Composition selon l'une des revendications précédentes, se présentant sous la forme d'un produit de soin et/ou de maquillage de la peau et/ou des lèvres et/ou des cils.

**39.** Composition selon l'une des revendications précédentes, se présentant sous la forme d'un fond de teint coulé, d'un fard à joues ou à paupières coulé, d'un rouge à lèvres, d'une base ou baume de soin pour les lèvres, d'un produit anti-cernes, d'un mascara ou d'un maquillage du corps.

**40.** Utilisation cosmétique d'au moins un copolymère d'oléfines cristallin ayant un taux de cristallinité au plus égal à 50 % et une masse molaire moyenne en poids $M_w \geq 30\ 000$, dans une composition cosmétique de maquillage de la peau et des lèvres ou de soin hygiénique du visage, du cou, des mains et du corps.

**41.** Utilisation d'au moins un copolymère d'oléfines cristallin ayant un taux de cristallinité au plus égal à 50 % et une masse molaire moyenne en poids $M_w \geq 30\ 000$, pour la fabrication d'une composition dermatologique ou pharmaceutique, destinée à être utilisée pour la protection, le traitement ou le soin du visage, du cou, des mains ou du corps.

**42.** Utilisation selon la revendication 40 ou 41, **caractérisé en ce que** le copolymère d'oléfines cristallin est choisi parmi :

(A) les copolymères d'$\alpha$-oléfines, les copolymères d'oléfines et de cyclooléfines, les copolymères d'$\alpha$-oléfines et de diénes ; et
(B) les copolymères d'$\alpha$-oléfines à motifs tactiques et atactiques.

**43.** Utilisation selon la revendication 42, **caractérisé en ce que** les copolymères sont choisis parmi les bipolymères d'éthylène ou de propylène et d'$\alpha$-oléfines en $C_4$-$C_{16}$, de préférence $C_4$-$C_{12}$, et les terpolymères d'éthylène, de propylène et d'$\alpha$-oléfines en $C_4$-$C_{16}$, de préférence $C_4$-$C_{12}$.

**44.** Utilisation selon la revendication 43, **caractérisé en ce que** l'$\alpha$-oléfine en $C_4$-$C_{16}$ est choisie parmi le butène-1, le pentène-1, l'hexène-1, l'heptène-1, l'octène-1, le nonène-1, le décène-1, l'undécène-1, le dodécène-1, le 3,3,5-triméthylhexène-1, le 3-méthylpentène-1 et le 4-méthylpentène-1.

**45.** Utilisation selon la revendication 42, **caractérisé en ce que** les copolymères d'oléfine et- de cyclooléfine sont choisis parmi les bipolymères d'éthylène ou de propylène avec le cyclobutène, le cyclohexène, le cyclooctadiène, le norbornène, le diméthane-octahydro-naphtalène, l'éthylènenorbornène, le vinylnorbornène et le 4-vinylcyclohexène et les terpolymères d'éthylène, de propylène et des cyclooléfines précédentes.

**46.** Utilisation selon la revendication 42, **caractérisé en ce que** les copolymères d'$\alpha$-oléfine et de diène sont choisis parmi les bipolymères éthylène/butadiène et éthylène/isoprène.

**47.** Utilisation selon la revendication 42, **caractérisé en ce que** les copolymères d'$\alpha$-oléfines à motifs tactiques et atactiques sont choisis parmi les polypropylènes à motifs isotactiques et atactiques et les polypropylènes à motifs syndiotactiques et atactiques.

**48.** Utilisation selon l'une quelconque des revendications 40 à 47, **caractérisé en ce que** le copolymère d'oléfines est obtenu par synthèse métallocène.

**Patentansprüche**

**1.** Zusammensetzungen zum Auftragen auf Keratinsubstanzen, die ein flüssige Fettphase und mindestens ein in der flüssigen Fettphase lösliches oder dispergierbares Copolymer in einer wirksamen Menge enthält,
**dadurch gekennzeichnet, dass**
das Copolymer ein Gewichtsmittel der Molmasse $\overline{M}_w$ von $M_w \geq 30\ 000$ aufweist und unter den kristallinen Olefin-Copolymeren mit einem Kristallinitätsgrad von höchstens 50 %, vorzugsweise 5 bis 40 % und noch besser 10 bis 35 % ausgewählt ist, wobei die Bereichsgrenzen eingeschlossen sind.

**2.** Zusammensetzung nach Anspruch 1, **dadurch gekennzeichnet, dass** das Copolymer mindestens 2 Gew.-%, bezogen auf das Gesamtgewicht der Zusammensetzung, ausmacht.

**3.** Zusammensetzung nach Anspruch 1 oder 2, **dadurch gekennzeichnet, dass** das Copolymer ein Gewichtsmittel der Molmasse $\overline{M}_w$ von $\overline{M}_w \geq 40\ 000$ aufweist.

**4.** Zusammensetzung nach Anspruch 3, **dadurch gekennzeichnet, dass** das Copolymer einen Polymolekularitätsindex $\frac{\overline{M}_w}{\overline{M}_n} \leq 3{,}5$ und vorzugsweise $\leq 2{,}5$ aufweist, wobei $\overline{M}_n$ das Zahlenmittel der Molmasse bedeutet.

5. Zusammensetzung nach einem der vorhergehenden Ansprüche, **dadurch gekennzeichnet, dass** das Copolymer ein bei Raumtemperatur festes Copolymer ist.

6. Zusammensetzung nach einem der vorhergehenden Ansprüche, **dadurch gekennzeichnet, dass** sie in Form eines gegossenen Produkts vorliegt, das eine kosmetische, dermatologische, hygienische oder pharmazeutische, flüssige Fettphase und mindestens ein festes Wachs enthält.

7. Zusammensetzung nach einem der vorhergehenden Ansprüche, **dadurch gekennzeichnet, dass** das Copolymer filmbildend ist.

8. Zusammensetzung nach Anspruch 7, **dadurch gekennzeichnet, dass** die flüssige Fettphase flüchtig ist.

9. Zusammensetzung nach Anspruch 8, **dadurch gekennzeichnet, dass** sie ferner mindestens einen Wirkstoff enthält, der unter den kosmetischen, dermatologischen, hygienischen oder pharmazeutischen Wirkstoffen und deren Gemischen ausgewählt ist.

10. Zusammensetzung nach einem der vorhergehenden Ansprüche, **dadurch gekennzeichnet, dass** sie ferner mindestens ein Farbmittel enthält.

11. Zusammensetzung nach einem der vorhergehenden Ansprüche, **dadurch gekennzeichnet, dass** das Copolymer von Olefinen ausgewählt ist unter:

(A) Copolymeren von α-Olefinen, Copolymeren von Olefinen und Cycloolefinen, Copolymeren von α-Olefinen und Monomeren mit einer oder mehreren ethylenischen Bindungen, wie Dienen; und
(B) Copolymeren von α-Olefinen mit taktischen und ataktischen Einheiten.

12. Zusammensetzung nach Anspruch 11, **dadurch gekennzeichnet, dass** die Copolymere von α-Olefinen unter den Bipolymeren von Ethylen oder Propylen und einem α-Olefin mit 4 bis 16 und vorzugsweise 4 bis 12 Kohlenstoffatomen und den Terpolymeren von Ethylen, Propylen und einem α-Olefin mit 4 bis 16 und vorzugsweise 4 bis 12 Kohlenstoffatomen ausgewählt sind.

13. Zusammensetzung nach Anspruch 12, **dadurch gekennzeichnet, dass** das C$_{4\text{-}16}$-α-Olefin unter 1-Buten, 1-Penten, 1-Hexen, 1-Hepten, 1-Octen, 1-Nonen, 1-Decen, 1-Undecen, 1-Dodecen, 3,3,5-Trimethyl-1-hexen, 3-Methyl-1-penten und 4-Methyl-1-penten ausgewählt ist.

14. Zusammensetzung nach Anspruch 12 oder 13, **dadurch gekennzeichnet, dass** der prozentuale Mengenanteil des α-Olefin höchstens 40 Mol-% beträgt und vorzugsweise bei 30 Mol-% oder darunter liegt.

15. Zusammensetzung nach Anspruch 11, **dadurch gekennzeichnet, dass** die Copolymere von Olefinen unter den Bipolymeren von Ethylen oder Propylen und Cyclobuten, Cyclohexen, Cyclooctadien, Norbornen, Dimethanooctahydronaphthalin (DMON), Ethylidennorbornen, Vinylnorbornen oder 4-Vinylcyclohexen und den Terpolymeren von Ethylen, Propylen und den genannten Cycloolefinen ausgewählt sind.

16. Zusammensetzung nach Anspruch 15, **dadurch gekennzeichnet, dass** das Olefin/Cycloolefin-Copolymer weniger als 20 Mol-% Cycloolefin enthält.

17. Zusammensetzung nach Anspruch 16, **dadurch gekennzeichnet, dass** das α-Olefin/Cycloolefin-Copolymer ein Ethylen/Norbornen-Copolymer ist, das weniger als 18 Mol-% Norbornen enthält.

18. Zusammensetzung nach Anspruch 11, **dadurch gekennzeichnet, dass** die Copolymere von α-Olefinen und Monomeren mit ethylenischer Doppelbindung (ethylenischen Doppelbindungen), wie Dienen, unter den Ethylen/Butadien-Bipolymeren und Ethylen/Isopren-Bipolymeren ausgewählt sind.

19. Zusammensetzung nach Anspruch 18, **dadurch gekennzeichnet, dass** das Bipolymer weniger als 20 Mol-% Monomere mit einer oder mehreren ethylenischen Doppelbindungen enthält.

20. Zusammensetzung nach Anspruch 11, **dadurch gekennzeichnet, dass** die Copolymere von α-Olefinen mit taktischen und ataktischen Einheiten unter den Polypropylenen mit isotaktischen und ataktischen Einheiten und den Polypropylenen mit syndiotaktischen und ataktischen Einheiten ausgewählt sind.

21. Zusammensetzung nach Anspruch 20, **dadurch gekennzeichnet, dass** der Gehalt an taktischen Einheiten unter 30 Mol-% liegt.

22. Zusammensetzung nach einem der vorhergehenden Ansprüche, **dadurch gekennzeichnet, dass** die Olefin-Copolymere durch Metallocen-Synthese hergestellt werden.

23. Zusammensetzung nach einem der vorhergehenden Ansprüche, wobei die flüssige Fettphase aus Ölen mineralischer, tierischer, pflanzlicher oder synthetischer Herkunft, Ölen auf Kohlenstoffbasis, Kohlenwasserstoffölen, fluorierten Ölen und/oder silicierten Ölen einzeln oder in Form von Gemischen dieser Öle besteht.

24. Zusammensetzung nach einem der vorhergehenden Ansprüche, wobei die flüssige Fettphase unter Paraffinöl, Vaselineöl, Nerzöl, Schildkrötenöl, Sojaöl, Perhydrosqualen, Süßmandelöl, Calophyllumöl, Palmöl, Parleam, Traubenkernöl, Sesamöl, Maisöl, Rapsöl, Sonnenblumenöl, Baumwollsamenöl, Aprikosenkernöl, Ricinusöl, Avocadoöl, Jojobaöl, Olivenöl oder Getreidekeimöl; Estern von Lanolinsäure, Ölsäure, Laurinsäure oder Stearinsäure; Fettsäureestern, wie Isopropylmyristat, Isopropylpalmitat, Butylstearat, Hexyllaurat, Diisopropyladipat, Isononylisononat, 2-Ethylhexylpalmitat, 2-Hexyldecyllaurat, 2-Octyldecylpalmitat, 2-Octyldodecylmyristat, 2-Octyldodecyllactat, 2-Diethylhexylsuccinat, Diisostearylmalat, Glyceryltriisostearat oder Diglyceryltriisostearat; höheren Fettsäuren mit mindestens 12 Kohlenstoffatomen, wie Myristinsäure, Palmitinsäure, Stearinsäure, Behensäure, Ölsäure, Linolsäure, Linolensäure oder Isostearinsäure; höheren Fettalkoholen mit mindestens 12 Kohlenstoffatomen, wie Stearylalkohol, Oleylalkohol, Linoleylalkohol oder Linolenylalkohol, Isostearylalkohol oder Octyldodecanol; Siliconölen, wie PDMS, die gegebenenfalls phenyliert sind, beispielsweise Phenyltrimethiconen, Phenyltrimethylsiloxydiphenylsiloxanen, Diphenylmethyldimethyltrisiloxanen, Diphenyldimethiconen, Phenyldimethiconen, Polymethylphenylsiloxanen, oder gegebenenfalls mit aliphatischen und/oder aromatischen Gruppen oder mit funktionalen Gruppen substituierten PDMS, wie Hydroxygruppen, Thiogruppen und/oder Aminogruppen; mit Fettsäuren, Fettalkoholen oder Polyoxyalkylenen modifizierten Polysiloxanen, fluorierten Siliconen, perfluorierten Ölen; flüchtigen Ölen, wie Octamethylcyclotetrasiloxan, Decamethylcyclopentasiloxan, Hexadecamethylcyclohexasiloxan, Heptamethylhexyltrisiloxan, Heptamethyloctyltrisiloxan oder $C_{8-16}$-Isoparaffinen und insbesondere Isododecan und Isohexadecan ausgewählt ist.

25. Zusammensetzung nach einem der vorhergehenden Ansprüche, wobei die flüssige Fettphase ausgewählt ist unter:

- nicht wässrigen flüssigen Verbindungen mit einem Gesamtsolubilitätsparameter im Solubilitätsraum nach HANSEN unter 17 $(MPa)^{1/2}$, oder
- Monoalkoholen mit einem Gesamtsolubilitätsparameter im Solubilitätsraum nach HANSEN von 20 $(MPa)^{1/2}$ oder darunter,
- oder deren Gemischen.

26. Zusammensetzung nach einem der vorhergehenden Ansprüche, wobei die Phase mindestens ein bei Umgebungstemperatur flüchtiges Öl enthält.

27. Zusammensetzung nach einem der vorhergehenden Ansprüche, die ferner mindestens eine zusätzliche Fettphase enthält, die unter den Wachsen, Gummis und/oder pastösen Fettsubstanzen pflanzlicher, tierischer, mineralischer, synthetischer oder silicierter Herkunft und deren Gemischen ausgewählt ist.

28. Zusammensetzung nach einem der vorhergehenden Ansprüche, **dadurch gekennzeichnet, dass** sie mindestens eine Verbindung in Pulverform enthält, die unter den Füllstoffen, Pigmenten, Perlglanzpigmenten und deren Gemischen ausgewählt ist.

29. Zusammensetzung nach Anspruch 28, **dadurch gekennzeichnet, dass** die pulverförmige Verbindung und das Copolymer in einem Verhältnis Pigmente/Copolymer unter 1 vorhanden sind.

30. Zusammensetzung nach Anspruch 28 oder 29, **dadurch gekennzeichnet, dass** die pulverförmige Verbindung 0,1 bis 98 % des Gesamtgewichts der Zusammensetzung ausmacht.

31. Zusammensetzung nach Anspruch 29, **dadurch gekennzeichnet, dass** die pulverförmige Zusammensetzung 1 bis 30 % des Gesamtgewichts der Zusammensetzung ausmacht.

32. Zusammensetzung nach einem der vorhergehenden Ansprüche, **dadurch gekennzeichnet, dass** das Olefin-Copolymer (als Trockensubstanz) bis zu 60 % des Gesamtgewichts der Zusammensetzung ausmacht.

33. Zusammensetzung nach einem der vorhergehenden Ansprüche, **dadurch gekennzeichnet, dass** das Olefin-Copolymer (als Trockensubstanz) 12 bis 60 % des Gesamtgewichts der Zusammensetzung ausmacht.

34. Zusammensetzung nach einem der vorhergehenden Ansprüche, **dadurch gekennzeichnet, dass** die flüssige Fettphase mindestens ein Öl enthält, das unter den $C_{8-16}$-Isoparaffinen, geradkettigen oder cyclischen Siliconen mit 2 bis 7 Siliciumatomen, wobei die Silicone gegebenenfalls Alkylgruppen mit 1 bis 10 Kohlenstoffatomen enthalten, und deren Gemischen ausgewählt ist.

35. Zusammensetzung nach einem der vorhergehenden Ansprüche, die als Stick oder Stift, in Form einer weichen Paste mit einer dynamischen Viskosität bei 25 °C in der Größenordnung von 1 bis 40 Pa·s, in Tiegelform, als öliges Gel; als ölige Flüssigkeit; oder als Vesikeldispersion, die ionische und/oder nichtionische Lipide enthält, vorliegt.

36. Zusammensetzung nach einem der Ansprüche 1 bis 35, **dadurch gekennzeichnet, dass** sie als Wasser-in-Öl-Emulsion, Öl-in-Wasser-Emulsion oder Wasser-in Wachs-Emulsion vorliegt, wobei das Wachs aus einem Gemisch von mindestens einem kristallinen Polymer von Olefinen mit einem Kristallinitätsgrad von höchstens 50 % und mindestens einem Öl bestehen kann.

37. Zusammensetzung nach einem der Ansprüche 1 bis 34, die in wasserfreier Form vorliegt.

38. Zusammensetzung nach einem der vorhergehenden Ansprüche, die als Produkt zur Pflege und/oder zum Schminken der Haut und/oder der Lippen und/oder der Wimpern vorliegt.

39. Zusammensetzung nach einem der vorhergehenden Ansprüche, die als gegossenes Make-up, gegossenes Wagenrouge, gegossener Lidschatten, Lippenstift, Lippenpflegebasis oder Lippenpflegebalsam, Produkt gegen Augenringe, Mascara oder Produkt zum Schminken des Körpers vorliegt.

40. Kosmetische Verwendung mindestens eines kristallinen Copolymers von Olefinen mit einem Kristallinitätsgrad von höchsten 50 % und einem Gewichtsmittel der Molmasse $M_w \geq 30\,000$ in einer kosmetischen Zusammensetzung zum Schminken der Haut und der Lippen oder zur Pflege des Gesichts, des Halses, der Hände und des Körpers.

41. Verwendung mindestens eines kristallinen Copolymers von Olefinen mit einem Kristallinitätsgrad von höchstens 50 % und einem Gewichtsmittel der Molmasse $M_w \geq 30\,000$ zur Herstellung einer dermatologischen oder pharmazeutischen Zusammensetzung, die dazu vorgesehen ist, für den Schutz, die Behandlung oder die Pflege des Gesichts, des Halses, der Hände oder des Körpers verwendet zu werden.

42. Verwendung nach Anspruch 40 oder 41, **dadurch gekennzeichnet, dass** das kristalline Copolymer von Olefinen ausgewählt ist unter:

(A) den Copolymeren von $\alpha$-Olefinen, den Copolymeren von Olefinen und Cycloolefinen und den Copolymeren von $\alpha$-Olefinen und Dienen; und

(B) den Copolymeren von $\alpha$-Olefinen mit taktischen und ataktischen Einheiten.

43. Verwendung nach Anspruch 42, **dadurch gekennzeichnet, dass** die Copolymere unter den Bipolymeren von Ethylen oder Propylen und $C_{4-16}$-$\alpha$-Olefinen und vorzugsweise $C_{4-12}$-$\alpha$-Olefinen und den Terpolymeren von Ethylen, Propylen und $C_{4-16}$-$\alpha$-Olefinen und vorzugsweise $C_{4-12}$-$\alpha$-Olefinen ausgewählt sind.

44. Verwendung nach Anspruch 43, **dadurch gekennzeichnet, dass** das $C_{4-16}$-$\alpha$-Olefin unter 1-Buten, 1-Penten, 1-Hexen, 1-Hepten, 1-Octen, 1-Nonen, 1-Decen, 1-Undecen, 1-Dodecen, 3,3,5-Trimethyl-1-hexen, 3-Methyl-1-penten und 4-Methyl-1-penten ausgewählt ist.

45. Verwendung nach Anspruch 42, **dadurch gekennzeichnet, dass** die Olefin/Cycloolefin-Copolymere unter den Bipolymeren von Ethylen oder Propylen mit Cyclobuten, Cyclohexen, Cyclooctadien, Norbornen, Dimethano-octahydronaphthalen, Ethylennorbornen, Vinylnorbornen und 4-Vinylcyclohexen und den Terpolymeren von Ethylen, Propylen und den genannten Cycloolefinen ausgewählt ist.

46. Verwendung nach Anspruch 42, **dadurch gekennzeichnet, dass** die $\alpha$-Olefin/Dien-Copolymere unter den Bipolymeren Ethylen/Butadien und Ethylen/Isopren ausgewählt sind.

47. Verwendung nach Anspruch 42, **dadurch gekennzeichnet, dass** die Copolymere von $\alpha$-Olefinen mit taktischen und ataktischen Einheiten unter den Polypropylenen mit isotaktischen und ataktischen Einheiten und den Polypropylenen mit syndiotaktischen und ataktischen Einheiten ausgewählt sind.

48. Verwendung nach einem der Ansprüche 40 bis 47, **dadurch gekennzeichnet, dass** das Copolymer von Olefinen durch Metallocen-Synthese hergestellt wird.

**Claims**

1. Composition for application to keratinous substances, comprising a liquid fatty phase and an effective amount of at least one copolymer which is soluble or dispersible in the liquid fatty phase, **characterized in that** the copolymer has a weight-average molar mass $\overline{M}_w$ such that $\overline{M}_w \geq 30\,000$ and is chosen from crystalline olefin copolymers having a degree of crystallinity at most equal to 50%, preferably of between 5 and 40% and better still of between 10 and 35%, limits included.

2. Composition according to Claim 1, **characterized in that** the copolymer represents at least 2% by weight with respect to the total weight of the composition.

3. Composition according to Claim 1 or 2, **characterized in that** the copolymer has a weight-average molar mass $\overline{M}_w$ such that $\overline{M}_w \geq 40\ 000$.

4. Composition according to Claim 3, **characterized in that** the copolymer has a polydispersity index $\overline{M}_w/\overline{M}_n \leq 3.5$, preferably $\leq 2.5$, where $\overline{M}_n$ is the number-average molar mass.

5. Composition according to any one of the preceding claims, **characterized in that** the copolymer is a copolymer which is solid at ambient temperature.

6. Composition according to any one of the preceding claims, **characterized in that** it is provided in the form of a cast product comprising a cosmetic, dermatological, hygiene or pharmaceutical liquid fatty phase and at least one solid wax.

7. Composition according to one of the preceding claims, **characterized in that** the copolymer can form a film.

8. Composition according to Claim 7, **characterized in that** the liquid fatty phase is volatile.

9. Composition according to Claim 8, **characterized in that** it additionally comprises at least one active principle chosen from cosmetic, dermatological, hygiene or pharmaceutical active principles and their mixtures.

10. Composition according to any one of the preceding claims, **characterized in that** it additionally comprises at least one colouring material.

11. Composition according to any one of the preceding claims, **characterized in that** the olefin copolymer is chosen from:

(A) copolymers of $\alpha$-olefins, copolymers of olefins and of cycloolefins or copolymers of $\alpha$-olefins and of monomers comprising ethylenic bond(s), such as dienes; and
(B) $\alpha$-olefin copolymers comprising tactic and atactic units.

12. Composition according to Claim 11, **characterized in that** the $\alpha$-olefin copolymers are chosen from bipolymers of ethylene or of propylene and of a $C_4$-$C_{16}$, preferably $C_4$-$C_{12}$, $\alpha$-olefin and terpolymers of ethylene, of propylene and of a $C_4$-$C_{16}$, preferably $C_4$-$C_{12}$, $\alpha$-olefin.

13. Composition according to Claim 12, **characterized in that** the $C_4$-$C_{16}$ $\alpha$-olefin is chosen from 1-butene, 1-pentene, 1-hexene, 1-heptene, 1-octene, 1-nonene, 1-decene, 1-undecene, 1-dodecene, 3,3,5-trimethyl-1-hexene, 3-methyl-1-pentene and 4-methyl-1-pentene.

14. Composition according to Claim 12 or 13, **characterized in that** the molar percentage of $\alpha$-olefin is at most equal to 40%, preferably equal to or less than 30%.

15. Composition according to Claim 11, **characterized in that** the olefin copolymers are chosen from bipolymers of ethylene or of propylene with cyclobutene, cyclohexene, cyclooctadiene, norbornene, dimethanooctahydronaphthalene (DMON), ethylidenenorbornene, vinylnorbornene and 4-vinylcyclohexene and terpolymers of ethylene, of propylene and of the preceding cycloolefins.

16. Composition according to Claim 15, **characterized in that** the copolymer of olefin and of cycloolefin comprises less than 20 mol% of cycloolefin.

17. Composition according to Claim 16, **characterized in that** the copolymer of $\alpha$-olefin and of cycloolefin is an ethylene/norbornene copolymer comprising less than 18 mol% of norbornene.

18. Composition according to Claim 11, **characterized in that** the copolymers of $\alpha$-olefin and of monomers comprising ethylenic bond(s), such as dienes, are chosen from ethylene/butadiene and ethylene/isoprene bipolymers.

19. Composition according to Claim 18, **characterized in that** the bipolymer comprises less than 20 mol% of monomers comprising ethylenic bond(s).

20. Composition according to Claim 11, **characterized in that** the $\alpha$-olefin copolymers comprising tactic and atactic units are chosen from polypropylenes comprising isotactic and atactic units and polypropylenes comprising syndiotactic and atactic units.

21. Composition according to Claim 20, **characterized in that** the level of tactic units is less than 30 mol%.

22. Composition according to any one of the preceding claims, **characterized in that** the olefin copolymers are obtained by metallocene synthesis.

23. Composition according to one of the preceding claims, in which the liquid fatty phase is composed of oils of mineral, animal, vegetable or synthetic origin, carbonaceous oils, hydrocarbonaceous oils, fluorinated oils and/or silicone oils, alone or as a mixture.

**24.** Composition according to one of the preceding claims, in which the liquid fatty phase is chosen from liquid paraffin or liquid petrolatum, mink oil, turtle oil, soybean oil, perhydrosqualene, sweet almond oil, calophyllum oil, palm oil, parleam oil, grape seed oil, sesame oil, maize oil, rapeseed oil, sunflower oil, cottonseed oil, apricot oil, castor oil, avocado oil, jojoba oil, olive oil or cereal germ oil; esters of lanolic acid, oleic acid, lauric acid or stearic acid; fatty esters, such as isopropyl myristate, isopropyl palmitate, butyl stearate, hexyl laurate, diisopropyl adipate, isononyl isononate, 2-ethylhexyl palmitate, 2-hexyldecyl laurate, 2-octyldecyl palmitate, 2-octyldodecyl myristate or lactate, di(2-ethylhexyl) succinate, diisostearyl malate, glyceryl triisostearate or diglyceryl triisostearate; higher fatty acids comprising at least 12 carbon atoms, such as myristic acid, palmitic acid, stearic acid, behenic acid, oleic acid, linoleic acid, linolenic acid or isostearic acid; higher fatty alcohols comprising at least 12 carbon atoms, such as stearyl alcohol or oleyl alcohol, linoleyl or linolenyl alcohol, isostearyl alcohol or octyldodecanol; silicone oils, such as PDMSs, which are optionally phenylated, such as phenyl trimethicones, phenyltrimethylsiloxydiphenylsiloxanes, diphenylmethyldimethyltrisiloxanes, diphenyl dimethicones, phenyl dimethicones or polymethylphenylsiloxanes, or which are optionally substituted by aliphatic and/or aromatic groups or by functional groups, such as hydroxyl, thiol and/or amine groups; polysiloxanes modified by fatty acids, fatty alcohols or polyoxyalkylenes, fluorinated silicones or perfluorinated oils; or volatile oils, such as octamethylcyclotetrasiloxane, decamethylcyclopentasiloxane, hexadecamethylcyclohexasiloxane, heptamethylhexyltrisiloxane, heptamethyloctyltrisiloxane or $C_8$-$C_{16}$ isoparaffins and in particular isododecane and isohexadecane.

**25.** Composition according to one of the preceding claims, in which the liquid fatty phase is chosen from the group consisting of:

- non-aqueous liquid compounds having an overall solubility parameter according to the Hansen solubility space of less than 17 $(MPa)^{1/2}$,
- or monoalcohols having an overall solubility parameter according to the Hansen solubility space of less than or equal to 20 $(MPa)^{1/2}$,
- or their mixtures.

**26.** Composition according to any one of the preceding claims, in which the phase comprises at least one oil which is volatile at ambient temperature.

**27.** Composition according to one of the preceding claims, furthermore comprising at least one additional fatty phase chosen from waxes, gums and/or pasty fatty substances of vegetable, animal, mineral or synthetic origin or silicone waxes, gums and/or pasty fatty substances, and their mixtures.

**28.** Composition according to any one of the preceding claims, **characterized in that** it comprises at least one pulverulent compound chosen from fillers, pigments, pearlescence agents and their mixtures.

**29.** Composition according to Claim 28, **characterized in that** the pulverulent compound and the copolymer are present in a pulverulent compound/copolymer ratio of less than 1.

**30.** Composition according to Claim 28 or 29, **characterized in that** the pulverulent compound represents from 0.1 to 98% of the total weight of the composition.

**31.** Composition according to Claim 29, **characterized in that** the pulverulent compound represents from 1 to 30% of the total weight of the composition.

**32.** Composition according to one of the preceding claims, **characterized in that** the olefin copolymer represents (as dry matter) up to 60% of the total weight of the composition.

**33.** Composition according to one of the preceding claims, **characterized in that** the olefin copolymer represents (as dry matter) from 12 to 60% of the total weight of the composition.

**34.** Composition according to one of the preceding claims, **characterized in that** the liquid fatty phase comprises at least one oil chosen from $C_8$-$C_{16}$ isoparaffins and linear or cyclic silicones having from 2 to 7 silicon atoms, these silicones optionally comprising alkyl groups having from 1 to 10 carbon atoms, and their mixtures.

**35.** Composition according to one of the preceding claims, which is provided in the form of a stick or tube; in the form of a soft paste with a dynamic viscosity at 25°C of the order of 1 to 40 Pa.s; in the form of a dish; in the form of an oily gel; in the form of an oily liquid; or in the form of a vesicular dispersion comprising ionic and/or nonionic lipids.

**36.** Composition according to any one of Claims 1 to 35, **characterized in that** it is provided in the form of a water-in-oil, oil-in-water or water-in-wax emulsion, it being possible for the said wax to be composed of a mixture of at least one crystalline olefin copolymer having a degree of crystallinity at most equal to 50% and at least one oil.

**37.** Composition according to any one of Claims 1 to 34, which is provided in the anhydrous form.

**38.** Composition according to one of the preceding claims, which is provided in the form of a product for caring for and/or making-up the skin and/or lips and/or eyelashes.

**39.** Composition according to one of the preceding claims, which is provided in the form of a cast foundation, of a cast face powder, of a cast eyeshadow, of a lipstick, of a care base or balm for the lips, of a concealer, of a mascara or of a make-up for the body.

**40.** Cosmetic use of at least one crystalline olefin copolymer having a degree of crystallinity at most equal to 50% and a weight-average molar mass $\overline{M}_w \geq 30\ 000$ in a cosmetic composition for making up the skin and lips or for the hygienic care of the face, neck, hands and body.

**41.** Use of at least one crystalline olefin copolymer having a degree of crystallinity at most equal to 50% and a weight-average molar mass $\overline{M}_w \geq 30\ 000$ in the manufacture of a dermatological or pharmaceutical composition intended to be used for the protection, treatment or care of the face, neck, hands or body.

**42.** Use according to Claim 40 or 41, **characterized in that** the crystalline olefin copolymer is chosen from:

(A) copolymers of $\alpha$-olefins, copolymers of olefins and of cycloolefins or copolymers of $\alpha$-olefins and of dienes; and
(B) $\alpha$-olefin copolymers comprising tactic and atactic units.

**43.** Use according to Claim 42, **characterized in that** the copolymers are chosen from bipolymers of ethylene or of propylene and of $C_9$-$C_{16}$, preferably $C_4$-$C_{12}$, $\alpha$-olefins and terpolymers of ethylene, of propylene and of $C_4$-$C_{16}$, preferably $C_9$-$C_{12}$, $\alpha$-olefins.

**44.** Use according to Claim 43, **characterized in that** the $C_4$-$C_{16}$ $\alpha$-olefin is chosen from 1-butene, 1-pentene, 1-hexene, 1-heptene, 1-octene, 1-nonene, 1-decene, 1-undecene, 1-dodecene, 3,3,5-trimethyl-1-hexene, 3-methyl-1-pentene and 4-methyl-1-pentene.

**45.** Use according to Claim 42, **characterized in that** the copolymers of olefin and cycloolefin are chosen from bipolymers of ethylene or of propylene with cyclobutene, cyclohexene, cyclooctadiene, norbornene, dimethanooctahydronaphthalene, ethylenenorbornene, vinylnorbornene and 4-vinylcyclohexene and terpolymers of ethylene, of propylene and of the preceding cyclolefins.

**46.** Use according to Claim 42, **characterized in that** the copolymers of $\alpha$-olefin and of diene are chosen from ethylene/butadiene and ethylene/isoprene bipolymers.

**47.** Use according to Claim 42, **characterized in that** the $\alpha$-olefin copolymers comprising tactic and atactic units are chosen from polypropylenes comprising isotactic and atactic units and polypropylenes comprising syndiotactic and atactic units.

**48.** Use according to any one of Claims 40 to 47, **characterized in that** the olefin copolymer is obtained by metallocene synthesis.